(19) Europäisches Patentamt
European Patent Office
Office européen des brevets

(11) EP 1 533 368 A1

(12) EUROPEAN PATENT APPLICATION
published in accordance with Art. 158(3) EPC

(43) Date of publication:
**25.05.2005 Bulletin 2005/21**

(21) Application number: **03738614.1**

(22) Date of filing: **01.07.2003**

(51) Int Cl.[7]: **C12N 5/10**, C12N 15/09, C12Q 1/02, A61K 45/00, A61P 3/08

(86) International application number:
**PCT/JP2003/008367**

(87) International publication number:
**WO 2004/005497 (15.01.2004 Gazette 2004/03)**

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **02.07.2002 JP 2002193814**

(71) Applicant: **YAMANOUCHI PHARMACEUTICAL CO. LTD.**
**Tokyo 103-8411 (JP)**

(72) Inventors:
- **OGINO, Makoto,**
**c/o Yamanouchi Pharmaceut. Co., Ltd**
**Tsukuba-shi, Ibaraki 305-8585 (JP)**
- **ENDOH, Hideki,**
**c/o Yamanouchi Pharmaceut. Co., Ltd**
**Tsukuba-shi, Ibaraki 305-8585 (JP)**

(74) Representative: **HOFFMANN - EITLE**
**Patent- und Rechtsanwälte**
**Arabellastrasse 4**
**81925 München (DE)**

(54) **METHOD OF SCREENING INSULIN RESISTANCE IMPROVING DRUG**

(57) A method for identifying and screening a novel substance promoting the transcription induction activity of PPARγ and improving insulin resistance by promoting the interaction between PPAR promoting the transcription induction activity of PPARγ and p68 RNA helicase and/or promoting the expression of p68RNA helicase is disclosed. The method is a new type method for screening an agent for improving insulin resistance by promoting the transcription induction activity of PPARγ which is different from the conventional PPAR agonist. Furthermore, the method for producing a pharmaceutical composition for improving insulin resistance which comprises the substance obtainable by the above method for screening as an active ingredient is disclosed.

EP 1 533 368 A1

SEQUENCE LISTING

<110> Yamanouchi Pharmaceutical Co.,Ltd.

<120> A screening method for detecting PPAR modulators using p68 RNA helicase

<130> Y0329-PCT

<150> JP2002-193814
<151> 2002-07-02

<160> 16

<170> PatentIn version 3.1

<210> 1
<211> 1845
<212> DNA
<213> Homo sapiens

<220>
<221> CDS
<222> (1)..(1845)
<223> Inventor: Ogino, Makoto, Endoh, Hideki

<400> 1
atg tcg ggt tat tcg agt gac cga gac cgc ggc cgg gac cga ggg ttt 48
Met Ser Gly Tyr Ser Ser Asp Arg Asp Arg Gly Arg Asp Arg Gly Phe
1 5 10 15

ggt gca cct cga ttt gga gga agt agg gca ggg ccc tta tct gga aag 96
Gly Ala Pro Arg Phe Gly Gly Ser Arg Ala Gly Pro Leu Ser Gly Lys
20 25 30

```
aag ttt gga aac cct ggg gag aaa tta gtt aaa aag aag tgg aat ctt      144
Lys Phe Gly Asn Pro Gly Glu Lys Leu Val Lys Lys Lys Trp Asn Leu
        35                  40                  45

gat gag ctg cct aaa ttt gag aag aat ttt tat caa gag cac cct gat      192
Asp Glu Leu Pro Lys Phe Glu Lys Asn Phe Tyr Gln Glu His Pro Asp
    50                  55                  60

ttg gct agg cgc aca gca caa gag gtg gaa aca tac aga aga agc aag      240
Leu Ala Arg Arg Thr Ala Gln Glu Val Glu Thr Tyr Arg Arg Ser Lys
65                  70                  75                  80

gaa att aca gtt aga ggt cac aac tgc ccg aag cca gtt cta aat ttt      288
Glu Ile Thr Val Arg Gly His Asn Cys Pro Lys Pro Val Leu Asn Phe
                85                  90                  95

tat gaa gcc aat ttc cct gca aat gtc atg gat gtt att gca aga cag      336
Tyr Glu Ala Asn Phe Pro Ala Asn Val Met Asp Val Ile Ala Arg Gln
            100                 105                 110

aat ttc act gaa ccc act gct att caa gct cag gga tgg cca gtt gct      384
Asn Phe Thr Glu Pro Thr Ala Ile Gln Ala Gln Gly Trp Pro Val Ala
        115                 120                 125

cta agt gga ttg gat atg gtt gga gtg gca cag act gga tct ggg aaa      432
Leu Ser Gly Leu Asp Met Val Gly Val Ala Gln Thr Gly Ser Gly Lys
    130                 135                 140

aca ttg tct tat ttg ctt cct gcc att gtc cac atc aat cat cag cca      480
Thr Leu Ser Tyr Leu Leu Pro Ala Ile Val His Ile Asn His Gln Pro
145                 150                 155                 160

ttc cta gag aga ggc gat ggg cct att tgt ttg gtg ctg gca cca act      528
Phe Leu Glu Arg Gly Asp Gly Pro Ile Cys Leu Val Leu Ala Pro Thr
                165                 170                 175
```

```
cgg gaa ctg gcc caa cag gtg cag caa gta gct gct gaa tat tgt aga      576
Arg Glu Leu Ala Gln Gln Val Gln Gln Val Ala Ala Glu Tyr Cys Arg
            180                 185                 190

gca tgt cgc ttg aag tct act tgt atc tac ggt ggt gct cct aag gga      624
Ala Cys Arg Leu Lys Ser Thr Cys Ile Tyr Gly Gly Ala Pro Lys Gly
        195                 200                 205

cca caa ata cgt gat ttg gag aga ggt gtg gaa atc tgt att gca aca      672
Pro Gln Ile Arg Asp Leu Glu Arg Gly Val Glu Ile Cys Ile Ala Thr
    210                 215                 220

cct gga aga ctg att gac ttt tta gag tgt gga aaa acc aat ctg aga      720
Pro Gly Arg Leu Ile Asp Phe Leu Glu Cys Gly Lys Thr Asn Leu Arg
225                 230                 235                     240

aga aca acc tac ctt gtc ctt gat gaa gca gat aga atg ctt gat atg      768
Arg Thr Thr Tyr Leu Val Leu Asp Glu Ala Asp Arg Met Leu Asp Met
                245                 250                 255

ggc ttt gaa ccc caa ata agg aag att gtg gat caa ata aga cct gat      816
Gly Phe Glu Pro Gln Ile Arg Lys Ile Val Asp Gln Ile Arg Pro Asp
            260                 265                 270

agg caa act cta atg tgg agt gcg act tgg cca aaa gaa gta aga cag      864
Arg Gln Thr Leu Met Trp Ser Ala Thr Trp Pro Lys Glu Val Arg Gln
        275                 280                 285

ctt gct gaa gat ttc ctg aaa gac tat att cat ata aac att ggt gca      912
Leu Ala Glu Asp Phe Leu Lys Asp Tyr Ile His Ile Asn Ile Gly Ala
    290                 295                 300

ctt gaa ctg agt gca aac cac aac att ctt cag att gtg gat gtg tgt      960
Leu Glu Leu Ser Ala Asn His Asn Ile Leu Gln Ile Val Asp Val Cys
305                 310                 315                     320
```

cat gac gta gaa aag gat gaa aaa ctt att cgt cta atg gaa gag atc 1008
His Asp Val Glu Lys Asp Glu Lys Leu Ile Arg Leu Met Glu Glu Ile
325 330 335

atg agt gag aag gag aat aaa acc att gtt ttt gtg gaa acc aaa aga 1056
Met Ser Glu Lys Glu Asn Lys Thr Ile Val Phe Val Glu Thr Lys Arg
340 345 350

aga tgt gat gag ctt acc aga aaa atg agg aga gat ggg tgg cct gcc 1104
Arg Cys Asp Glu Leu Thr Arg Lys Met Arg Arg Asp Gly Trp Pro Ala
355 360 365

atg ggt atc cat ggt gac aag agt caa caa gag cgt gac tgg gtt cta 1152
Met Gly Ile His Gly Asp Lys Ser Gln Gln Glu Arg Asp Trp Val Leu
370 375 380

aat gaa ttc aaa cat gga aaa gct cct att ctg att gct aca gat gtg 1200
Asn Glu Phe Lys His Gly Lys Ala Pro Ile Leu Ile Ala Thr Asp Val
385 390 395 400

gcc tcc aga ggg cta gat gtg gaa gat gtg aaa ttt gtc atc aat tat 1248
Ala Ser Arg Gly Leu Asp Val Glu Asp Val Lys Phe Val Ile Asn Tyr
405 410 415

gac tac cct aac tcc tca gag gat tat att cat cga att gga aga act 1296
Asp Tyr Pro Asn Ser Ser Glu Asp Tyr Ile His Arg Ile Gly Arg Thr
420 425 430

gct cgc agt acc aaa aca ggc aca gca tac act ttc ttt aca cct aat 1344
Ala Arg Ser Thr Lys Thr Gly Thr Ala Tyr Thr Phe Phe Thr Pro Asn
435 440 445

aac ata aag caa gtg agc gac ctt atc tct gtg ctt cgt gaa gct aat 1392
Asn Ile Lys Gln Val Ser Asp Leu Ile Ser Val Leu Arg Glu Ala Asn
450 455 460

caa gca att aat ccc aag ttg ctt cag ttg gtc gaa gac aga ggt tca 1440
Gln Ala Ile Asn Pro Lys Leu Leu Gln Leu Val Glu Asp Arg Gly Ser
465 470 475 480

ggt cgt tcc agg ggt aga gga ggc atg aag gat gac cgt cgg gac aga 1488
Gly Arg Ser Arg Gly Arg Gly Gly Met Lys Asp Asp Arg Arg Asp Arg
485 490 495

tac tct gcg ggc aaa agg ggt gga ttt aat acc ttt aga gac agg gaa 1536
Tyr Ser Ala Gly Lys Arg Gly Gly Phe Asn Thr Phe Arg Asp Arg Glu
500 505 510

aat tat gac aga ggt tac tct agc ctg ctt aaa aga gat ttt ggg gca 1584
Asn Tyr Asp Arg Gly Tyr Ser Ser Leu Leu Lys Arg Asp Phe Gly Ala
515 520 525

aaa act cag aat ggt gtt tac agt gct gca aat tac acc aat ggg agc 1632
Lys Thr Gln Asn Gly Val Tyr Ser Ala Ala Asn Tyr Thr Asn Gly Ser
530 535 540

ttt gga agt aat ttt gtg tct gct ggt ata cag acc agt ttt agg act 1680
Phe Gly Ser Asn Phe Val Ser Ala Gly Ile Gln Thr Ser Phe Arg Thr
545 550 555 560

ggt aat cca aca ggg act tac cag aat ggt tat gat agc act cag caa 1728
Gly Asn Pro Thr Gly Thr Tyr Gln Asn Gly Tyr Asp Ser Thr Gln Gln
565 570 575

tac gga agt aat gtt cca aat atg cac aat ggt atg aac caa cag gca 1776
Tyr Gly Ser Asn Val Pro Asn Met His Asn Gly Met Asn Gln Gln Ala
580 585 590

tat gca tat cct gct act gca gct gca cct atg att ggt tat cca atg 1824
Tyr Ala Tyr Pro Ala Thr Ala Ala Ala Pro Met Ile Gly Tyr Pro Met
595 600 605

cca aca gga tat tcc caa taa 1845
Pro Thr Gly Tyr Ser Gln
610

<210> 2
<211> 614
<212> PRT
<213> Homo sapiens

<400> 2

Met Ser Gly Tyr Ser Ser Asp Arg Asp Arg Gly Arg Asp Arg Gly Phe
1 5 10 15

Gly Ala Pro Arg Phe Gly Gly Ser Arg Ala Gly Pro Leu Ser Gly Lys
20 25 30

Lys Phe Gly Asn Pro Gly Glu Lys Leu Val Lys Lys Lys Trp Asn Leu
35 40 45

Asp Glu Leu Pro Lys Phe Glu Lys Asn Phe Tyr Gln Glu His Pro Asp
50 55 60

Leu Ala Arg Arg Thr Ala Gln Glu Val Glu Thr Tyr Arg Arg Ser Lys
65 70 75 80

Glu Ile Thr Val Arg Gly His Asn Cys Pro Lys Pro Val Leu Asn Phe
85 90 95

Tyr Glu Ala Asn Phe Pro Ala Asn Val Met Asp Val Ile Ala Arg Gln
100 105 110

Asn Phe Thr Glu Pro Thr Ala Ile Gln Ala Gln Gly Trp Pro Val Ala
115 120 125

Leu Ser Gly Leu Asp Met Val Gly Val Ala Gln Thr Gly Ser Gly Lys
130 135 140

Thr Leu Ser Tyr Leu Leu Pro Ala Ile Val His Ile Asn His Gln Pro
145 150 155 160

Phe Leu Glu Arg Gly Asp Gly Pro Ile Cys Leu Val Leu Ala Pro Thr
165 170 175

Arg Glu Leu Ala Gln Gln Val Gln Gln Val Ala Ala Glu Tyr Cys Arg
180 185 190

Ala Cys Arg Leu Lys Ser Thr Cys Ile Tyr Gly Gly Ala Pro Lys Gly
195 200 205

Pro Gln Ile Arg Asp Leu Glu Arg Gly Val Glu Ile Cys Ile Ala Thr
210 215 220

Pro Gly Arg Leu Ile Asp Phe Leu Glu Cys Gly Lys Thr Asn Leu Arg
225 230 235 240

Arg Thr Thr Tyr Leu Val Leu Asp Glu Ala Asp Arg Met Leu Asp Met
245 250 255

Gly Phe Glu Pro Gln Ile Arg Lys Ile Val Asp Gln Ile Arg Pro Asp
260 265 270

Arg Gln Thr Leu Met Trp Ser Ala Thr Trp Pro Lys Glu Val Arg Gln
275 280 285

Leu Ala Glu Asp Phe Leu Lys Asp Tyr Ile His Ile Asn Ile Gly Ala
290 295 300

Leu Glu Leu Ser Ala Asn His Asn Ile Leu Gln Ile Val Asp Val Cys
305 310 315 320

His Asp Val Glu Lys Asp Glu Lys Leu Ile Arg Leu Met Glu Glu Ile
325 330 335

Met Ser Glu Lys Glu Asn Lys Thr Ile Val Phe Val Glu Thr Lys Arg
340 345 350

Arg Cys Asp Glu Leu Thr Arg Lys Met Arg Arg Asp Gly Trp Pro Ala
355 360 365

Met Gly Ile His Gly Asp Lys Ser Gln Gln Glu Arg Asp Trp Val Leu
370 375 380

Asn Glu Phe Lys His Gly Lys Ala Pro Ile Leu Ile Ala Thr Asp Val
385 390 395 400

Ala Ser Arg Gly Leu Asp Val Glu Asp Val Lys Phe Val Ile Asn Tyr
405 410 415

Asp Tyr Pro Asn Ser Ser Glu Asp Tyr Ile His Arg Ile Gly Arg Thr
420 425 430

Ala Arg Ser Thr Lys Thr Gly Thr Ala Tyr Thr Phe Phe Thr Pro Asn
435 440 445

Asn Ile Lys Gln Val Ser Asp Leu Ile Ser Val Leu Arg Glu Ala Asn
450 455 460

Gln Ala Ile Asn Pro Lys Leu Leu Gln Leu Val Glu Asp Arg Gly Ser
465 470 475 480

Gly Arg Ser Arg Gly Arg Gly Gly Met Lys Asp Asp Arg Arg Asp Arg
485 490 495

Tyr Ser Ala Gly Lys Arg Gly Gly Phe Asn Thr Phe Arg Asp Arg Glu
500 505 510

Asn Tyr Asp Arg Gly Tyr Ser Ser Leu Leu Lys Arg Asp Phe Gly Ala
515 520 525

Lys Thr Gln Asn Gly Val Tyr Ser Ala Ala Asn Tyr Thr Asn Gly Ser
530 535 540

Phe Gly Ser Asn Phe Val Ser Ala Gly Ile Gln Thr Ser Phe Arg Thr
545 550 555 560

Gly Asn Pro Thr Gly Thr Tyr Gln Asn Gly Tyr Asp Ser Thr Gln Gln
565 570 575

Tyr Gly Ser Asn Val Pro Asn Met His Asn Gly Met Asn Gln Gln Ala
580 585 590

Tyr Ala Tyr Pro Ala Thr Ala Ala Ala Pro Met Ile Gly Tyr Pro Met
595 600 605

Pro Thr Gly Tyr Ser Gln
610

<210> 3
<211> 1518
<212> DNA
<213> Homo sapiens

<220>
<221> CDS
<222> (1)..(1518)
<223>

<400> 3
atg ggt gaa act ctg gga gat tct cct att gac cca gaa agc gat tcc 48

Met Gly Glu Thr Leu Gly Asp Ser Pro Ile Asp Pro Glu Ser Asp Ser
1 5 10 15

ttc act gat aca ctg tct gca aac ata tca caa gaa atg acc atg gtt 96
Phe Thr Asp Thr Leu Ser Ala Asn Ile Ser Gln Glu Met Thr Met Val
20 25 30

gac aca gag atg cca ttc tgg ccc acc aac ttt ggg atc agc tcc gtg 144
Asp Thr Glu Met Pro Phe Trp Pro Thr Asn Phe Gly Ile Ser Ser Val
35 40 45

gat ctc tcc gta atg gaa gac cac tcc cac tcc ttt gat atc aag ccc 192
Asp Leu Ser Val Met Glu Asp His Ser His Ser Phe Asp Ile Lys Pro
50 55 60

ttc act act gtt gac ttc tcc agc att tct act cca cat tac gaa gac 240
Phe Thr Thr Val Asp Phe Ser Ser Ile Ser Thr Pro His Tyr Glu Asp
65 70 75 80

att cca ttc aca aga aca gat cca gtg gtt gca gat tac aag tat gac 288
Ile Pro Phe Thr Arg Thr Asp Pro Val Val Ala Asp Tyr Lys Tyr Asp
85 90 95

ctg aaa ctt caa gag tac caa agt gca atc aaa gtg gag cct gca tct 336
Leu Lys Leu Gln Glu Tyr Gln Ser Ala Ile Lys Val Glu Pro Ala Ser
100 105 110

cca cct tat tat tct gag aag act cag ctc tac aat aag cct cat gaa 384
Pro Pro Tyr Tyr Ser Glu Lys Thr Gln Leu Tyr Asn Lys Pro His Glu
115 120 125

gag cct tcc aac tcc ctc atg gca att gaa tgt cgt gtc tgt gga gat 432
Glu Pro Ser Asn Ser Leu Met Ala Ile Glu Cys Arg Val Cys Gly Asp
130 135 140

aaa gct tct gga ttt cac tat gga gtt cat gct tgt gaa gga tgc aag 480

Lys Ala Ser Gly Phe His Tyr Gly Val His Ala Cys Glu Gly Cys Lys
145 150 155 160

ggt ttc ttc cgg aga aca atc aga ttg aag ctt atc tat gac aga tgt 528
Gly Phe Phe Arg Arg Thr Ile Arg Leu Lys Leu Ile Tyr Asp Arg Cys
165 170 175

gat ctt aac tgt cgg atc cac aaa aaa agt aga aat aaa tgt cag tac 576
Asp Leu Asn Cys Arg Ile His Lys Lys Ser Arg Asn Lys Cys Gln Tyr
180 185 190

tgt cgg ttt cag aaa tgc ctt gca gtg ggg atg tct cat aat gcc atc 624
Cys Arg Phe Gln Lys Cys Leu Ala Val Gly Met Ser His Asn Ala Ile
195 200 205

agg ttt ggg cgg atg cca cag gcc gag aag gag aag ctg ttg gcg gag 672
Arg Phe Gly Arg Met Pro Gln Ala Glu Lys Glu Lys Leu Leu Ala Glu
210 215 220

atc tcc agt gat atc gac cag ctg aat cca gag tcc gct gac ctc cgg 720
Ile Ser Ser Asp Ile Asp Gln Leu Asn Pro Glu Ser Ala Asp Leu Arg
225 230 235 240

gcc ctg gca aaa cat ttg tat gac tca tac ata aag tcc ttc ccg ctg 768
Ala Leu Ala Lys His Leu Tyr Asp Ser Tyr Ile Lys Ser Phe Pro Leu
245 250 255

acc aaa gca aag gcg agg gcg atc ttg aca gga aag aca aca gac aaa 816
Thr Lys Ala Lys Ala Arg Ala Ile Leu Thr Gly Lys Thr Thr Asp Lys
260 265 270

tca cca ttc gtt atc tat gac atg aat tcc tta atg atg gga gaa gat 864
Ser Pro Phe Val Ile Tyr Asp Met Asn Ser Leu Met Met Gly Glu Asp
275 280 285

aaa atc aag ttc aaa cac atc acc ccc ctg cag gag cag agc aaa gag 912

Lys Ile Lys Phe Lys His Ile Thr Pro Leu Gln Glu Gln Ser Lys Glu
290 295 300

gtg gcc atc cgc atc ttt cag ggc tgc cag ttt cgc tcc gtg gag gct 960
Val Ala Ile Arg Ile Phe Gln Gly Cys Gln Phe Arg Ser Val Glu Ala
305 310 315 320

gtg cag gag atc aca gag tat gcc aaa agc att cct ggt ttt gta aat 1008
Val Gln Glu Ile Thr Glu Tyr Ala Lys Ser Ile Pro Gly Phe Val Asn
325 330 335

ctt gac ttg aac gac caa gta act ctc ctc aaa tat gga gtc cac gag 1056
Leu Asp Leu Asn Asp Gln Val Thr Leu Leu Lys Tyr Gly Val His Glu
340 345 350

atc att tac aca atg ctg gcc tcc ttg atg aat aaa gat ggg gtt ctc 1104
Ile Ile Tyr Thr Met Leu Ala Ser Leu Met Asn Lys Asp Gly Val Leu
355 360 365

ata tcc gag ggc caa ggc ttc atg aca agg gag ttt cta aag agc ctg 1152
Ile Ser Glu Gly Gln Gly Phe Met Thr Arg Glu Phe Leu Lys Ser Leu
370 375 380

cga aag cct ttt ggt gac ttt atg gag ccc aag ttt gag ttt gct gtg 1200
Arg Lys Pro Phe Gly Asp Phe Met Glu Pro Lys Phe Glu Phe Ala Val
385 390 395 400

aag ttc aat gca ctg gaa tta gat gac agc gac ttg gca ata ttt att 1248
Lys Phe Asn Ala Leu Glu Leu Asp Asp Ser Asp Leu Ala Ile Phe Ile
405 410 415

gct gtc att att ctc agt gga gac cgc cca ggt ttg ctg aat gtg aag 1296
Ala Val Ile Ile Leu Ser Gly Asp Arg Pro Gly Leu Leu Asn Val Lys
420 425 430

ccc att gaa gac att caa gac aac ctg cta caa gcc ctg gag ctc cag 1344

Pro Ile Glu Asp Ile Gln Asp Asn Leu Leu Gln Ala Leu Glu Leu Gln
435 440 445

ctg aag ctg aac cac cct gag tcc tca cag ctg ttt gcc aag ctg ctc 1392
Leu Lys Leu Asn His Pro Glu Ser Ser Gln Leu Phe Ala Lys Leu Leu
450 455 460

cag aaa atg aca gac ctc aga cag att gtc acg gaa cac gtg cag cta 1440
Gln Lys Met Thr Asp Leu Arg Gln Ile Val Thr Glu His Val Gln Leu
465 470 475 480

ctg cag gtg atc aag aag acg gag aca gac atg agt ctt cac ccg ctc 1488
Leu Gln Val Ile Lys Lys Thr Glu Thr Asp Met Ser Leu His Pro Leu
485 490 495

ctg cag gag atc tac aag gac ttg tac tag 1518
Leu Gln Glu Ile Tyr Lys Asp Leu Tyr
500 505

<210> 4
<211> 505
<212> PRT
<213> Homo sapiens

<400> 4

Met Gly Glu Thr Leu Gly Asp Ser Pro Ile Asp Pro Glu Ser Asp Ser
1 5 10 15

Phe Thr Asp Thr Leu Ser Ala Asn Ile Ser Gln Glu Met Thr Met Val
20 25 30

Asp Thr Glu Met Pro Phe Trp Pro Thr Asn Phe Gly Ile Ser Ser Val

35 40 45

Asp Leu Ser Val Met Glu Asp His Ser His Ser Phe Asp Ile Lys Pro
50 55 60

Phe Thr Thr Val Asp Phe Ser Ser Ile Ser Thr Pro His Tyr Glu Asp
65 70 75 80

Ile Pro Phe Thr Arg Thr Asp Pro Val Val Ala Asp Tyr Lys Tyr Asp
85 90 95

Leu Lys Leu Gln Glu Tyr Gln Ser Ala Ile Lys Val Glu Pro Ala Ser
100 105 110

Pro Pro Tyr Tyr Ser Glu Lys Thr Gln Leu Tyr Asn Lys Pro His Glu
115 120 125

Glu Pro Ser Asn Ser Leu Met Ala Ile Glu Cys Arg Val Cys Gly Asp
130 135 140

Lys Ala Ser Gly Phe His Tyr Gly Val His Ala Cys Glu Gly Cys Lys
145 150 155 160

Gly Phe Phe Arg Arg Thr Ile Arg Leu Lys Leu Ile Tyr Asp Arg Cys
165 170 175

Asp Leu Asn Cys Arg Ile His Lys Lys Ser Arg Asn Lys Cys Gln Tyr

180 185 190

Cys Arg Phe Gln Lys Cys Leu Ala Val Gly Met Ser His Asn Ala Ile
195 200 205

Arg Phe Gly Arg Met Pro Gln Ala Glu Lys Glu Lys Leu Leu Ala Glu
210 215 220

Ile Ser Ser Asp Ile Asp Gln Leu Asn Pro Glu Ser Ala Asp Leu Arg
225 230 235 240

Ala Leu Ala Lys His Leu Tyr Asp Ser Tyr Ile Lys Ser Phe Pro Leu
245 250 255

Thr Lys Ala Lys Ala Arg Ala Ile Leu Thr Gly Lys Thr Thr Asp Lys
260 265 270

Ser Pro Phe Val Ile Tyr Asp Met Asn Ser Leu Met Met Gly Glu Asp
275 280 285

Lys Ile Lys Phe Lys His Ile Thr Pro Leu Gln Glu Gln Ser Lys Glu
290 295 300

Val Ala Ile Arg Ile Phe Gln Gly Cys Gln Phe Arg Ser Val Glu Ala
305 310 315 320

Val Gln Glu Ile Thr Glu Tyr Ala Lys Ser Ile Pro Gly Phe Val Asn

325 330 335

Leu Asp Leu Asn Asp Gln Val Thr Leu Leu Lys Tyr Gly Val His Glu
340 345 350

Ile Ile Tyr Thr Met Leu Ala Ser Leu Met Asn Lys Asp Gly Val Leu
355 360 365

Ile Ser Glu Gly Gln Gly Phe Met Thr Arg Glu Phe Leu Lys Ser Leu
370 375 380

Arg Lys Pro Phe Gly Asp Phe Met Glu Pro Lys Phe Glu Phe Ala Val
385 390 395 400

Lys Phe Asn Ala Leu Glu Leu Asp Asp Ser Asp Leu Ala Ile Phe Ile
405 410 415

Ala Val Ile Ile Leu Ser Gly Asp Arg Pro Gly Leu Leu Asn Val Lys
420 425 430

Pro Ile Glu Asp Ile Gln Asp Asn Leu Leu Gln Ala Leu Glu Leu Gln
435 440 445

Leu Lys Leu Asn His Pro Glu Ser Ser Gln Leu Phe Ala Lys Leu Leu
450 455 460

Gln Lys Met Thr Asp Leu Arg Gln Ile Val Thr Glu His Val Gln Leu

465 470 475 480

Leu Gln Val Ile Lys Lys Thr Glu Thr Asp Met Ser Leu His Pro Leu
485 490 495

Leu Gln Glu Ile Tyr Lys Asp Leu Tyr
500 505

<210> 5
<211> 1300
<212> DNA
<213> Homo sapiens

<220>
<221> promoter
<222> (1)..(1300)
<223>

<400> 5
ccctcagggc ccatagcgca agggcggagg gcacacggac agcggctaga cgcccccacag 60

aaagacaagt ccggggacga cccttctgac cgctcttttt acagccagga cccaagtgtc 120

ctaccggcct cgccccagtg cctctctctc tcccacagca tactgctgtt ccacggcctc 180

gaagcgaaga ggtggtgaag ctgagagacc ctatccaggg aacccgccag cgcgacgcgg 240

cgtctgaagg tcacgagccc tgccgacagc ccagacccag tccgggctag cccgaggcct 300

ccctggaggt ggacggtttc agtccacaca tactgggacc ccagggagac actcaccagc 360

atccgagcct gccatgtttc agaggcaggt cgccgccgga ctccgacgcg gccgggaagg 420

cgacggtgtc ctggaaggac cgatccacgc agacccgaca ctggggcgcg gacgcacgaa 480

ccaaagcgcg gggaaggagg cgtgaaagaa ggacggacgt taaaagagct tctcgccgct 540

gattggtcat cagaggagca cttcctttca caggacgtga aacgggggcg gtttggggaa 600

gtttagagac cattctccgc cgaccaaaac ccgtcaaagg attatcagac acgcgggtcg 660

gacggtccac atcagccggc agcccgggcg ggtcccgggg tgcgagcagc gcacttccgg 720

tgagctattt cgttttgtat ccctccgccg acgtcaacgg gaaagtagtg cggaccgctc 780

tctcggtggt ccggggtggt acagccacgt gacaacgcca ggccccgcct tccccctctt 840

ttggttacag acgtgagggc tctttggaga cgtaaacatc tccgagtggc gagggtgggc 900

ggggctgggc ttgggaaagg gcggggtggc ttgcttgagg tgtggaaaga ccagaagaag 960

gtgaggtcaa gagagtgcag aatgaggcat tccaatggtg ggtgggccct gacctgagag 1020

agtggcgcgg ggaggggtga aagcgcggcg atcctggaac gccagcgggc gttgcggcct 1080

atgcgcgagg ggcggggcga ttaggtcata gagcggctcc cagcgttccc tgcggcgtag 1140

gaggcggtcc agactataaa agcggctgcc ggaaagcggc cggcacctca ttcatttcta 1200

ccggtctcta gtagtgcagc ttcggctggt gtcatcggtg tccttcctcc gctgccgccc 1260

ccgcaaggct tcgccgtcat cgaggccatt tccagcgact 1300

<210> 6
<211> 47
<212> DNA
<213> Artificial

<220>
<223> Description of Artificial Sequence:an artificially synthesized primer sequence

<400> 6
agacagttga ctgtatcgga attcatgggt gaaactctgg gagattc 47

<210> 7
<211> 30
<212> DNA
<213> Artificial

<220>
<223> Description of Artificial Sequence:an artificially synthesized primer sequence

<400> 7
aggagctcct agtacaagtc cttgtagatc 30

<210> 8
<211> 46
<212> DNA
<213> Artificial

<220>
<223> Description of Artificial Sequence:an artificially synthesized primer sequence

<400> 8
gcgaagaagt ccaaagcggc cgctatgcaa ggcatttctg aaaccg 46

<210> 9
<211> 29

<212> DNA
<213> Homo sapiens

<400> 9
ggagagatgg gtggcctgcc atgggtatc 29

<210> 10
<211> 41
<212> DNA
<213> Artificial

<220>
<223> Description of Artificial Sequence:an artificially synthesized primer sequence

<400> 10
cttctagatc ttattgggaa tatcctgttg gcattggata a 41

<210> 11
<211> 18
<212> DNA
<213> Mus musculus

<400> 11
gcacagcagg tgcagcaa 18

<210> 12
<211> 24
<212> DNA
<213> Mus musculus

<400> 12
gcaccaccat agatgcaagt agac 24

<210> 13
<211> 20
<212> DNA
<213> Mus musculus

<400> 13
aaagtggaga ttgttgccat 20

<210> 14
<211> 19
<212> DNA
<213> Mus musculus

<400> 14
ttgactgtgc cgttgaatt 19

<210> 15
<211> 26
<212> DNA
<213> Artificial

<220>
<223> Description of Artificial Sequence:an artificially synthesized primer sequence

<400> 15
agggtaccct cagggcccat agcgca 26

<210> 16
<211> 26
<212> DNA

<213> Artificial

<220>
<223> Description of Artificial Sequence:an artificially synthesized primer sequence

<400> 16
agctcgagtc gctggaaatg gcctcg 26

**Description**

Technical Field

The present invention relates to a substance promoting the transcription induction activity of PPARγ, and/or a method for screening an agent for improving insulin resistance.

Background of the Invention

A thiazolidine derivative which is recognized by its effect as an agent for improving insulin resistance is shown to have function as an agonist of peroxisome proliferator activated receptor gamma (PPARγ) (Lehmann, *et al*., *J. Biol. Chem*. Vol. 270, pp. 12953-12956, 1995). It has been known that PPARγ belongs to the nuclear receptor super family and binds to a response element in upstream region of a target gene as a transcriptional activation factor which is activated via ligand binding to induce the transcription (Mangelsdorf, *et al*., *Cell,* Vol. 83, pp. 835-839, 1995). PPARγ agonists were reported to arrest cellular proliferation and promote cellular differentiation (Kitamura, *et al*., *Jpn. J. Cancer Res.,* Vol. 90, Item 75, 1999). The expression of PPARγ is especially recognized in fat tissues (Tontonoz, *et al., Genes and Development,* Vol.8, pp. 1224-1234, 1994; Tontonoz, *et al*., *Cell,* Vol.79, pp. 1147-1156, 1994). The induction of the differentiation of fat cells is not occurred in homozygous PPARγ-deficient mice. Additionally, administration of thiazolidine derivatives acting as PPARγ agonists decrease the number of large fat cells and increase the number of small fat cells (Kubota, *et al., Mol. Cell,* Vol.4, pp. 597-609, 1999). Based on the above findings, the mechanism of the improving insulin resistance by thiazolidine derivatives is considered as follows. As the result of rapid promotion of fat cell differentiation by the PPARγ agonists, the generation of TNFα as the causative factor inducing insulin resistance is suppressed, while the expression of glucose transporter is promoted in peripheral tissues and the generation of free fatty acids is suppressed. As a result, glucose uptake into cells is activated to improve hyperglycemia (Lehmann, *et al., J. Biol. Chem*., Vol. 270, pp. 12953-12956, 1995). Since the affinity of thiazolidine derivatives with PPARγ has a correlation with the hypoglycemic activity *in vivo*, the activity of the compound group for improving insulin resistance is considered to be the activity mediated by PPARγ activation (Willson, *et al*., *J. Med. Chem*., Vol.39, pp.665-668, 1996). These have suggested that the promotion of the transcription induction activity of PPARγ improves insulin resistance and that the method for detecting PPARγ agonist is therefore an effective method for screening a therapeutic agent for improving insulin-resistant diabetes.

However, from recent clinical findings using thiazolidine derivatives, conventional synthetic ligands which have the PPARγ agonistic activity not only have the activity to improve insulin resistance but also cause disorders of liver functions without exception and additionally increase circulating plasma volume *in vivo* to trigger edema (see non-patent reference 1, non-patent reference 2 and non-patent reference 3). Since the disorders of liver functions induced by the synthetic PPARγ agonists are serious side effects and edema triggered is a very serious side effect causing cardiomegaly, the detachment of the main activity, namely insulin-resistance improvement from such serious side effects has been strongly desired. However, the molecular mechanism of the induction of such side effects by thiazolidine derivatives has not yet been elucidated.

Generally, nuclear receptors have two transcriptional activation domains in the structure. The N-terminal domain is called as AF-1 while the C-terminal domain is called as AF-2. Since it is reported that the AF-2 is involved in the transcriptional activation depending on the ligand (Mangelsdorf, *et al., Cell*, Vol. 83, pp. 841-850, 1995), a great number of research works have been carried out so far and have also been used for screening the agonists and the like. With regard to the AF-1, alternatively, not many findings exist other than the finding that the AF-1 relates to the ligand-independent transcriptional activation. In recent years, nonetheless, it is reported that a characteristic phenotype in some of humans with a point mutation in the AF-1 of PPARγ exists and that a human with the mutation of the 12-th proline into alanine is particularly more resistant to obesity compared with the wild type and shows good insulin sensitivity (see non-patent reference 4).

The transcription induction activity of PPARγ requires an interaction with transcriptional cofactors like other nuclear receptors and attempts have been carried out to identify a factor interactive with PPARγ. Actually, the binding of PPARγ with existing nuclear receptor-interactive factors has been examined, and plural molecules such as SRC-1 (Zhu, *et al*., *Gene Expr.* Vol.6, pp. 185-195, 1996) and CBP/p300 (Gelman, *et al., J. Biol. Chem.,* Vol.274, pp.7681-7688, 1999) are reported to be interactive with PPARγ. However, the group of these cofactors is believed to mainly bind to the AF-2, while what has been known as a cofactor binding to the AF-1 until now is only PGC-2 (Castillo, *et al., EMBO J.,* Vol. 18, pp. 3676-3687, 1999).

The nucleotide sequence and amino acid sequence of p68 RNA helicase are registered on a database (genpept X52104, genpept X15729, genpept BC016027, genpept AF015812). The upstream nucleotide sequence thereof is described in the non-patent reference 5. Additionally, molecules highly homologous with p68 RNA helicase are described in the patent reference 1, the patent reference 2, the patent reference 3 and the patent reference 4, which describe that the molecules which relates to wound healing and are useful as tumor markers. Meanwhile, it is demonstrated that p68 RNA helicase is a transcriptional coactivator binding to the AF-1 of estrogen receptor α as one of nuclear receptors (see non-patent reference 6). More recently, further, the possibility that p68 RNA helicase relates to the differentiation of fat cells has been indicated (see non-patent reference 7 and non-patent reference 8). However, the detailed molecular mechanism thereof has not yet been elucidated.

(Patent reference 1)
Pamphlet of International Publication No. 02/28999
(Patent reference 2)
Specification of Canada No. 2325226
(Patent reference 3)
Pamphlet of International Publication No. 01/60860
(Patent reference 4)
Pamphlet of International Publication No. 01/64707
(Non-patent reference 1)
*The Lancet,* (USA), 2000, Vol. 355,
(Non-patent reference 2)
*Diabetes Frontier*, 1999, Vol.10, p.811-818
(Non-patent reference 3)
*Diabetes Frontier*, 1999, Vol. 10, p. 819-824
(Non-patent reference 4)
*Nature Genetics,* (USA), 1998, Vol. 20, p.284-287
(Non-patent reference 5)
*Nucleic Acids Research*, (UK), 2000, Vol.28, p.932-939
(Non-patent reference 6)
Molecular and Cellular Biology, (USA), 1999, Vol.19, p.5363-5372
(Non-patent reference 7)
*Biochemical and Biophysical Research Communications,* (USA), 2001, vol.287, p. 435-439
(Non-patent reference 8)
*Animal Genetics,* (UK), 2000, Vol.31, p. 166-170

Disclosure of the Invention

The inventors of the present invention identified p68 RNA helicase as a protein binding to the AF-1 of PPARγ and found that p68 RNA helicase was expressed in human fat tissues. Further, it was found that the transcription induction activity of PPARγ is promoted when p68 RNA helicase is expressed too much. Subsequently, it was found that pioglitazone which is an agent for improving insulin resistance induced the expression of p68 RNA helicase and that the increase of the protein led to the improvement of diabetic conditions. Additionally, it was found that a region regulating the transcription in a suppressive manner by the analysis of the upstream region of p68 RNA helicase gene. More additionally, it was found that the effect point for the activation of PPARγ transcription by pioglitazone never removed the transcription suppressive regulation and that a substance elevating the expression level of p68 RNA helicase and a substance improving insulin resistance unlike conventional drugs improving insulin resistance could be detected and/or screened for by screening substances which has activity of removing the suppressive regulation of p68 RNA helicase gene.

Based on these findings, it was constructed that a method for identifying and screening a new substance promoting the transcription induction activity of PPARγ to improve insulin resistance by promoting the interaction between PPARγ and p68 RNA helicase and/or increase the expression of p68 RNA helicase. Thus, a method for screening a new type of an agent for improving insulin resistance which is different from conventional PPARγ agonists is provided by promoting the transcription induction activity of PPARγ, as well as a method for producing a pharmaceutical composition for improving insulin resistance. Thus, the present invention has been achieved.

Specifically, the present invention relates to those described below:

[1] A cell transformed by

i) a polynucleotide encoding a polypeptide which comprises an amino acid sequence represented by SEQ ID NO:2 in which 1 to 10 amino acids are deleted, substituted and/or inserted and which interacts with PPARγ,
ii) a polynucleotide encoding a fusion protein comprising at least the AF-1 of the PPARγ protein represented by SEQ ID NO:4 and the DNA binding domain of a transcription factor, and

iii) a reporter gene fused to a response element to which the DNA binding domain of said transcription factor can bind; or

a cell transformed by

i) a polynucleotide encoding a polypeptide comprising an amino acid sequence represented by SEQ ID NO: 2 in which 1 to 10 amino acids are deleted, substituted and/or inserted and which interacts with PPARγ and
ii) a reporter gene fused to a response element to which the PPARγ protein represented by SEQ ID NO:4 is able to bind, and expressing

a) a polypeptide comprising a protein consisting of an amino acid sequence represented by SEQ ID NO: 2 in which 1 to 10 amino acids are deleted, substituted, and/or inserted, and which interacts with PPARγ and b) the PPARγ protein represented by SEQ ID NO:4.

[2] The cell according to [1], wherein the transcription factor is a yeast GAL4 protein.
[3] The cell according to [1], wherein the reporter gene is a luciferase gene.
[4] A method for detecting whether or not a test substance promotes the transcription induction activity of PPARγ, comprising

i) a step of allowing the cell according to one of [1] to [3] to contact with the test substance, and
ii) a step of analyzing the change of the test substance-dependent interaction or the change of the test substance-dependent transcription induction activity of PPARγ, in which expression of the reporter gene is used as an index.

[5] A method for screening a substance promoting the transcription induction activity of PPARγ, comprising

i) a step of allowing the cell according to one of [1] to [3] to contact with a test substance,
ii) a step of analyzing the change of the test substance-dependent interaction or the change of the test substance-dependent transcription induction activity of PPARγ, in which expression of the reporter gene is used as an index and
iii) a step of selecting a test substance which activates the reporter activity.

[6] The method for screening according to [5], wherein the substance promoting the transcription induction activity of PPARγ is an agent for improving insulin resistance.
[7] A method for screening an agent for improving insulin resistance, comprising

i) a step of allowing a cell expressing PPAR-interactive p68 RNA helicase to contact with a test substance, and
ii) a step of analyzing the change of the test substance-dependent expression level of PPAR-interactive p68 RNA helicase.

[8] A screening method for an agent for improving insulin resistance, comprising

i) a step of allowing a cell transformed with a reporter gene fused with the promoter region of p68 RNA helicase represented by SEQ ID NO:5 to contact with a test substance, and
ii) a step of analyzing the change of the test substance-dependent transcription induction activity, in which the expression of the reporter gene is used as an index.

[9] A method for producing a pharmaceutical composition for improving insulin resistance, comprising
a screening step using the screening method according to one of [5] to [8], and
a formulation step using a substance obtainable by said screening.

Although in patent reference 1, it is described that a molecule highly homologous with p68 RNA helicase and names of numerous diseases for which the molecule relates to, it is not described that the relation of p68 RNA helicase with insulin resistance and the relation of p68 RNA helicase with PPARγ. It is said that a molecule highly homologous with p68 RNA helicase which is described in the patent reference 2 relates to wound healing. Additionally, it is described that a molecule highly homologous with p68 RNA helicase described in the patent references 3 or 4 is useful as a tumor marker and relates to various tumors. In none of the patent reference, it is described the relation of p68 RNA helicase or highly homologous molecules thereof with insulin resistance or PPARγ.

Therefore, it is a novel finding found by the inventors of the present invention that p68 RNA helicase binds to the AF-1 of PPARγ and act as its transcriptional coactivator. Additionally, a method for identifying and screening a new substance promoting the transcription induction activity of PPARγ to improve insulin resistance by promoting the interaction between PPARγ and p68 RNA helicase and/or inducing the expression of p68 RNA helicase, and a method for producing a pharmaceutical composition for improving insulin resistance are inventions achieved for the first time by the inventors of the present invention.

Brief Description of the Drawings

Fig. 1 is a graph which shows the luciferase activity in Example 2, where the luciferase activity is shown in the vertical axis and the amount of an expression vector of p68 RNA helicase is shown in the horizontal axis; and
Fig. 2 is a graph which shows the luciferase activity in Example 5 (3), where the luciferase activity is shown in the vertical axis and the co-transfected plasmid is shown in the horizontal axis. The diagonal bar shows the results without reagent addition and the black bar shows the results with reagent addition.

Best Mode for Carrying out the Invention

The present invention is now described in detail herein below.

[1] Cell of the present invention

The polypeptide consisting of the amino acid sequence represented by SEQ ID NO:2 is the known natural type p68 RNA helicase derived from humans. The polypeptide consisting of the amino acid sequence represented by SEQ ID NO:4 is the known natural type PPARγ derived from humans.
Polypeptides interactive with PPARγ for preparing the cell of the present invention for testing PPARγ transcription activity include:

(1) a polypeptide consisting of the amino acid sequence represented by SEQ ID NO:2;
(2) a polynucleotide encoding a polypeptide which comprises an amino acid sequence represented by SEQ ID No:2 in which 1 to 10 amino acids are deleted, substituted and/or inserted and which interacts with the AF-1 of PPARγ (called functionally equivalent variant hereinafter); and
(3) a polypeptide comprising a protein consisting of an amino acid sequence with 90% or more homology with the amino acid sequence represented by SEQ ID NO:2 and interacting with the AF-1 of PPARγ (called homologous peptide hereinafter).

The functionally equivalent variant includes "a polypeptide which comprises the amino acid sequence represented by SEQ ID NO:2 and which is a protein interactive with the AF-1 of PPARγ" and "a polypeptide which comprises an amino acid sequence represented by SEQ ID NO:2 in which 1 to 10, preferably 1 to 7, more preferably 1 to 5 amino acids are deleted, substituted and/or inserted and which is a protein interactive with the AF-1 of PPARγ".
The homologous peptide is not particularly limited as far as it consists of an amino acid sequence with 90% or more homology with the amino acid sequence represented by SEQ XD NO:2 and is a protein which interacts with AF-1 of PPARγ. The homologous peptide consists of an amino acid sequence has homology with preferably 90% or more, more preferably with 95% or more, further more preferably with 98% or more with the amino acid sequence represented by SEQ ID NO:2 and is preferably a protein interactive with the AF-1 of PPARγ. In the present specification, the term "homology" means the value Identities obtained by using the default parameters prepared by screening on the Clustal program (Higgins & Sharp, *Gene*, Vol.73, pp. 237-244, 1998; Thompson, *et al*., *Nucleic Acid Res.,* Vol.22, pp. 4673-4680, 1994). The parameters are defined as follows.
As pair wise alignment parameters, those described below are listed.
K tuple 1
Gap Penalty 3
Window 5
Diagonals Saved 5.
The polypeptide consisting of the amino acid represented by SEQ ID NO.:2, functionally equivalent variants thereof and homologous polypeptides thereof are collectively referred to as "PPAR-interactive p68 RNA helicase" hereinafter.
The gene encoding the PPARγ-fused protein for preparing the cell of the present invention for the PPARγ transcription induction test may be any gene encoding a fused protein consisting of at least the AF-1 of the PPARγ protein represented by SEQ ID NO:4 and the DNA binding domain of a transcription factor. The AF-1 of the PPARγ is a region represented by the nucleotide sequence at the first to 504-th positions of the nucleotide sequence represented by SEQ ID NO:3. As the DNA binding domain, the DNA binding domain of any transcription factor may be used. "DNA binding domain " is a domain functioning for binding to DNA and means such domain having a DNA binding potency to a response element but never having the transcription induction potency by itself.

In the mode for carrying out the present invention, the "transcription factor" for use of detecting the transcription induction ability of PPARγ is not limited as far as it is a transcription factor from eukaryote which have a domain binding to a specific DNA sequence in cellular nuclei. Further, the DNA binding domain of such transcription factor may be the one which has a DNA binding ability to a response element but does not have any transcription induction ability by itself. Such transcription factor includes for example yeast GAL4 protein (Keegan, *et al., Science,* Vol.231, p. 699-704, 1986; Ma, *et al*., *Cell*, Vol. 48, p. 847-853, 1987). The DNA binding domain and transcription induction domain of the GAL4 transcription factor exist for example on the N-terminal side (a domain containing amino acids, approximately at position 1 to position 147) in case of GAL4.

As the "response element", a DNA sequence is used, to which the DNA binding domain of a transcription factor is capable of binding. The region may be scissored out from the upstream region of the gene or the sequence may be chemically synthesized, for use. More detailed definition and examples of such "response element" are described in the fourth edition of "*Molecular Cell Biology* (Bunshi Saibo Seibutsu-gaku)", (translated by Maruyama *et al*., Tokyo Kagaku Dojin, 2001).

The "reporter gene" to be arranged downstream of the response element is not specifically limited as far as it is a reporter gene for general use. As such genes, enzyme encoding genes which can be assayed easily are preferable. The reporter gene includes for example chloramphenicol acetyltransferase gene (CAT) from bacteria transposon, luciferase gene (Luc) from firefly, and green fluorescence protein gene (GFP) from jellyfish. As the reporter gene, a gene which is functionally ligated to the downstream of the response element or a gene in which a response element is inserted in a promoter is used.

Polynucleotides encoding PPARγ, the DNA binding domain of a transcription factor, and the PPAR-interactive p68 RNA helicase can be isolated from cDNA libraries, by the screening of the polymerase chain reaction (PCR) or hybridization, using primers and probes designed and synthesized on the basis of the information of known amino acid sequences and nucleotide sequences. The PPAR-interactive p68 RNA helicase may be derived from any species as far as it is identified as the counterpart and interacts with PPARγ to effect on the transcription induction ability of the receptor in the presence of a ligand thereof. The PPAR-interactive p68 RNA helicase includes for example those from mammalian animals, such as humans (GenBank Accession No. X15729, X52104 and AF015812), mouse (GenBank Accession No. X65627), and lynx (GenBank accession No. AF110009). PPARγ includes but is not limited to any PPARγ from any species, as far as it can be identified as the same molecular species and can play the biological functions as a nuclear receptor. PPARγ includes for example those derived from mammalian animals, for example humans (GenBank Accession No. U79012), mouse (GenBank Accession No. U09138), and rat (GenBank Accession No. AB019561). Additionally, in PPARγ, there are two isoform types, namely PPARγ1 and PPARγ2. Compared with PPARγ2, PPARγ1 lacks the 30 amino acids in the N-terminal side. The remaining amino acid sequence is totally the same and is known that both of them are expressed in fat tissues.

A polynucleotide encoding PPARγ, the DNA binding domain of a transcription factor, or the PPAR-interactive p68 RNA helicase can be obtained for example in the following ways. However, these can be obtained not only by the following method but by the known procedures in "*Molecular Cloning*" [Sambrook, J., *et al*., Cold Spring Harbor Laboratory Press, 1989] as well.

For example, the following methods can be listed: (1) the PCR method; (2) a method using routine genetic engineering technique (in other words, a method for selecting a transformant strain containing desired amino acids from transformant strains obtained by transformation with cDNA libraries); or (3) a chemical synthesis method. The each production method can be carried out as described in WO 01/34785.

By the PCR method, for example, the polynucleotide described in this specification can be produced by procedures described in "the Mode for Carrying out the Invention", 1) Production method of protein gene a) First production method of the patent reference mentioned above. In the description, the term "human cell or tissue with an ability of generating the protein of the present invention" includes for example human fat tissue. A mRNA is extracted from human fat tissues. Then, the mRNA is subjected to reverse-transcriptase reaction in the presence of random primer or oligo dT primer, to synthesize a first cDNA chain. Using the resulting first cDNA chain and two primer types directed for a partial region of the intended gene by polymerase chain reaction (PCR) was carried out to obtain the polynucleotide of the present invention as a whole or as a part. More specifically, the polynucleotide of the present invention can be produced for example by the method described in Example 1.

By the method using routine genetic engineering technique, for example, a polynucleotide encoding PPARγ, the DNA binding domain of a transcription factor, or the PPAR-interactive p68 RNA helicase in this specification can be produced by the procedures described in "the Mode for Carrying out the Invention", 1) Production method of protein gene a) Second production method of the patent reference mentioned above.

By the method using chemical synthesis, for example, a polynucleotide encoding PPARγ, the DNA binding domain of a transcription factor, or the PPAR-interactive p68 RNA helicase in this specification can be produced by the methods described in "the Mode for Carrying out the Invention", 1) Production method of protein gene c) Third production method and d) Fourth production method of the patent reference mentioned above.

By the method described in "*Molecular Cloning*" [Sambrook, J., *et al*., Cold Spring Harbor Laboratory Press, 1989], DNAs encoding these individual regions are used singly or are ligated together, for conjugation to the downstream of an appropriate promoter to construct an expression system of PPARγ and PPAR-interactive p68 RNA helicase in test cells. Specifically, the polynucleotide thus obtained may be integrated in an appropriate vector plasmid and then be utilized with transfection into a host cell. These may be constructed so that the two may be included in one plasmid or the two may be included separately in individually different plasmids. Otherwise, a cell with such construction integrated in the chromosomal DNA may be obtained and then be used.

The reporter gene ligated to a response element is used by being constructed by general gene recombination techniques; integrating the construct in a vector plasmid; and transfecting the recombinant plasmid into a host cell. Otherwise, the reporter gene can be used after obtaining a cell in which such substitution is integrated in the chromosomal DNA.

PPARγ may be externally introduced. In case when a cell in which endogenous PPARγ is expressed abundantly is used as a host cell, for example in case when a cell is a fat-derived cell, among constitutions mentioned above, only a construct consisting of a reporter ligated to a response element and the PPAR-interactive p68 RNA helicase excluding PPARγ may be introduced.

More specifically, a fragment containing the isolated polynucleotide is again integrated in an appropriate vector plasmid and is able to thereby transform eukaryotic or prokaryotic host cells. By further inserting an appropriate promoter and a sequence involved in gene expression into such vectors, a gene can be expressed in the individual host cells. As a method for transforming host cell to express gene, for example, the method described in "the Mode for Carrying out the Invention", 2) Method for producing vector, host cell and recombinant protein of the present invention in the patent reference above can be used. An expression vector is not limited as far as the it carries a desired polynucleotide. For example, an expression vector obtained by inserting a desired polynucleotide into a known expression vector selected appropriately which is dependent on the host cell used can be cited.

The cell of the present invention can be obtained by co-transfection of a desired host cell with the said expression vector. More specifically, for example, a desired polynucleotide is integrated in an expression vector pcDNA3.1 for mammalian cells as described in Example 2, to obtain an expression vector for a desired protein, which is incorporated in COS-1 cells using a commercially available transfection reagent Lipofectamine 2000 to produce the transformant cell of the present invention.

The desired transformant cell obtained above can be cultured by routine methods and a desired protein is produced by the culture. As the culture medium used in the culture, various culture medium routinely used according to the employed host cell can be selected appropriately. For the COS-1 cells, for example, the Dulbecco's modified Eagle's minimum essential culture medium supplemented for example with the serum component of fetal bovine serum (FBS) and additionally supplemented with G418 may be used.

[2] Detection and screening method of the invention

The method of the present invention for identifying and screening a novel substance improving insulin resistance through the promotion of the transcription induction activity of PPARγ by promoting the interaction between PPARγ and the PPAR-interactive p68 RNA helicase or by inducing the expression of the PPAR-interactive p68 RNA helicase are described below.

The cell of the present invention (called testing cell hereinafter) is cultured in the presence of a test substance to detect and assay the promotion of the promoting activity of the PPAR-interactive p68 RNA helicase for PPARγ transcription induction ability via the expression of the reporter gene.

Additionally when the test substance induces the expression of PPAR-interactive p68 RNA helicase or suppresses the degradation of PPAR-interactive p68 RNA helicase, the increase of the expressed reporter activity is observed. Such substance is identified as a promoting agent for the transcription induction activity of PPARγ.

Any of them has a structure different from those of conventional PPAR agonists, and is expected to act as an agent for improving insulin resistance with the stronger main activity dissociating from side effects.

<Method for detecting and/or screening a substance promoting the transcription induction activity of PPARγ and a substance improving insulin resistance>

One mode for carrying out the present invention is a method for selecting and screening a substance promoting the transcription induction activity of PPARγ and a substance improving insulin resistance, including a step of allowing a test substance to contact with (1) a cell (testing cell) transformed with i) a polynucleotide encoding the PPAR-interactive p68 RNA helicase, ii) a polynucleotide encoding a fusion protein consisting of at least of the AF-1 of the PPARγ protein and the DNA binding domain of a transcription factor, and iii) a reporter gene fused to a response element to which the DNA binding domain of said transcription factor is able to bind, or 2) a cell transformed by i) a polynucleotide encoding the PPAR-interactive p68 RNA helicase and ii) a reporter gene fused to a response element to which the PPARγ protein is able to bind, and expressing a) PPAR-interactive p68 RNA helicase and b) the PPARγ protein to detect and assay the change of the promoting activity of the transcription activating ability of PPARγ with the test substance in the testing cell, using the expression of the reporter gene as an index.

One hybrid system is a method for detecting the protein-protein interaction using the expression of reporter gene as a marker. Generally, transcription factor has functionally different two regions of DNA binding domain and transcription activating domain. In order to examine the interaction between two types of proteins of X and Y by the one hybrid system, two types of 1) a fusion protein consisting of the DNA binding domain of transcription factor and X and 2) Y are simultaneously expressed in a culture cell. When the proteins of X and Y interact together, these form one transcription complex, which binds to the response element (specifically binding DNA site) of the transcription factor in the cell nucleus to activate the transcription of the reporter gene arranged in the downstream thereof. As described above, the interaction of two proteins can be replaced for the expression of the reporter gene, and can be detected. More specifically, the detection can be carried out by the method of Castillo, *et al*. (*EMBO J.,* Vol. 18, pp. 3676-3687, 1999). Thus, the activity of test substance on the interaction between PPARγ and the PPAR-interactive p68 RNA helicase can be replaced for the expression of the reporter gene for assay. A substance promoting the interaction between the PPAR-interactive p68 RNA helicase and PPARγ (namely, a substance promoting the transcription induction activity of PPARγ) and a substance improving insulin resistance can be detected and/or screened for. When PPARγ expressed in the cell used in the method for detecting and/or screening a substance promoting the PPARγ transcription induction activity and a substance improving insulin resistance is the full-length PPARγ protein, preferably, a PPARγ ligand is added to the assay system. Since it is reported that as the result of the change of the tertiary structure of nuclear receptor due to the ligand binding to the AF-2, transcriptional cofactors are recruited into the AF-1 and AF-2 to occur activation of transcription. More specifically, the screening can be carried out by the method described in Example 2.

The preferable PPARγ ligand to be added in using the full-length PPARγ is any PPARγ ligand may be used as far as it is capable of triggering the transcription induction ability of PPARγ and includes such ligand capable of triggering the transcription induction ability of PPARγ, at a final concentration of for example 1 to 1,000 nM, preferably 1 to 100 nM and more preferably 1 to 30 nM. The PPARγ ligand includes for example thiazolidine derivatives such as pioglitazone (Lehmann, *et al., J. Biol.* Vol. 270, pp. 12953-12956, 1995).

Another mode of the method including assaying the activity of a test substance on the interaction between PPARγ and the PPAR-interactive p68 RNA helicase is for example a biochemical assay method. By such method, binding between the PPAR-interactive p68 RNA helicase labeled with for example RI and a fusion protein consisting of an appropriate tag protein such as glutathione-S-transferase (GST), protein A, β-galactosidase, and maltose-binding protein (MBP) and the AF-1 of PPARγ is directly detected in the presence of test substance. More specifically, the method can be carried out by procedures described in Example 1.

Additionally, still another method is an immunochemical method (ELISA). In such a method, for example, in order to examine the interaction between two types of proteins of X and Y, X is preliminarily immobilized, Y and a test substance are mixed. Subsequently, the resulting mixture is washed by an appropriate method so as to remove non-specific binding and an antibody specifically reacting with Y by an antigen-antibody reaction is added. The amount of Y bound to the immobilized X can be replaced with the amount of the antibody specifically reacting with Y and can be detected. Using this, a substance promoting the interaction between the PPAR-interactive p68 RNA helicase and PPARγ and a substance improving insulin resistance can be detected and/or screened for.

The method for detecting and/or screening a substance promoting the transduction induction ability of PPARγ and a substance improving insulin resistance includes the methods described above. The PPAR for use in the mode may be either of 1) the AF-1, and 2) the full-length PPAR preferably together with ligand addition.

<Method for screening an agent for improving insulin resistance including a step of analyzing the change of the expression of the PPAR-interactive p68 RNA helicase>

i) An agent for improving insulin resistance can be screened for by a method of which feature is including a step of allowing a test substance in contact to a cell expressing the PPAR-interactive p68 RNA helicase to contact with a test substance, and ii) a step of analyzing the change of the expression level of the PPAR-interactive p68 RNA helicase which is dependent on the test substance.

The "cell" may be any cell as far as it expresses the PPAR-interactive p68 RNA helicase or a cell obtained by transforming an expression vector of the PPAR-interactive p68 RNA helicase. Preferably, the cell is a culture cell 3T3L1 described in Example 4 is cited. Whether or not the "cell expressing the PPAR-interactive p68 RNA helicase" expresses p68 RNA helicase can be identified by Northern blotting using a gene having the nucleotide sequence encoding p68 RNA helicase or a part thereof or by Western blotting using an antibody specific to p68 RNA helicase. With or without adding a test substance to the cells expressing the PPAR-interactive p68 RNA helicase, the cells are collected after culturing for a certain period. The change of the expression level of the PPAR-interactive p68 RNA helicase depending on the test substance can be assayed as the change of the amount of mRNA as a transcription product of the gene or the change of the amount of a protein encoded by the mRNA. By comparing the change of the expression level between in case of the with or without the addition of the test substance, the change of the expression level of the PPAR-interactive p68 RNA helicase which is dependent on the test substance can be analyzed. From the harvested cells, RNA or a cell extract solution can be obtained. The amount of the mRNA in the PPAR-interactive p68 RNA helicase in the recovered RNA can be detected for example by real-time PCR. More specifically, the screening can be carried out by the method described in Example 4. Additionally, the amount of the protein of PPAR-interactive p68 RNA helicase in the harvested cell extract solution can be detected for example by immunochemical methods (Western blotting, *etc.*). An agent for improving insulin resistance can be screened for by analyzing of the change of the expression level of the PPAR-interactive p68 RNA helicase.

<Method for screening an agent for improving insulin resistance using promoter of the PPAR-interactive p68 RNA helicase>

An agent for improving insulin resistance can be screened for by i) a step of allowing a cell transformed with a reporter gene fused to the promoter region of p68 RNA helicase-consisting of the nucleotide sequence represented by SEQ ID NO:5 to contact with a test substance and ii) a step of analyzing the change of the transcription induction activity which depends on the test substance, using the expression of the reporter gene as an index. The reporter gene assay (Tamura, *et al*., *Transcription Factor Research Method,* Yodosha, 1993) is a method for assaying the regulation of gene expression using the expression of a reporter gene as an index. Generally, gene expression is regulated with a part called promoter region existing in the 5'-upstream region thereof. The gene expression level at the stage of transcription can be estimated by assaying the activity of the promoter. When a test substance activates a promoter, the transcription of the reporter gene fused in downstream of the promoter region is activated. In such manner, the promoter-activating activity, namely the expression promoting activity can be replaced by the expression of the reporter gene and be detected. Thus, the activity of a test substance on the regulation of the expression of the PPAR-interactive p68 RNA helicase can be replaced by the expression of the reporter gene and detected by the reporter gene assay using promoter region of p68 RNA helicase. The "reporter gene" fused to the promoter region p68 RNA helicase consisting of the nucleotide sequence represented by SEQ ID NO:5 is not limited as far as it is used generally, and preferably, the reporter gene encodes enzyme which can be assayed quantitatively. For example, the reporter gene includes chloramphenicol acetyltransferase gene (CAT) derived from bacteria transposon, luciferase gene (Luc) derived from firefly and green fluorescence protein gene (GFP) derived from jellyfish. The reporter gene may be fused functionally to the promoter region of p68 RNA helicase consisting of the nucleotide sequence represented by SEQ ID NO:5. By comparing the expression level of the reporter gene between in case of contact with or without a test substance, the change of the transcription induction activity depending on the test substance can be analyzed. By carrying out the steps, screening a substance activating the expression of the PPAR-interactive p68 RNA helicase and a substance improving insulin resistance can be carried out. Specifically, said screening can be carried out by the method described in Example 5.

The test substance used in the screening method of the present invention is not particularly limited and includes for example commercially available compounds (including peptides), various known compounds (including peptides) registered in the chemical files, a group of compounds obtained by the combinatorial chemistry technique (Terrett, *et al*., J. Steele, *Tetrahedron*, Vol. 51, pp. 8135-8173, 1995), bacterial culture supernatants, natural components derived from plants and marine organisms, animal tissue extracts or chemically and biologically modified compounds (including peptides) of compounds (including peptides) selected by the screening method of the present invention.

[3] Method for producing a pharmaceutical composition for improving insulin resistance

The present invention includes a method for producing a pharmaceutical composition for improving insulin resistance, of which feature is including a screening step using the screening method of the present invention and a formulation step using a substance obtained by the screening described above.
The formulation containing the substance obtained by the screening method of the present invention as the active component can be prepared, using carriers, excipients and/or other additives for general use in the formulation of the active component, depending on the type of the active component.
The administration includes oral administration via tablets, pills, capsules, granules, fine granules, powders or oral liquids, or parenteral administration via injections intravenous and intramuscular injections or injections into joints, suppositories, transcutaneous administration preparations or transmucosal administration preparations. For peptides to be digested in stomach, in particular, parenteral administration such as intravenous injection is preferable.
A solid composition for oral administration contains one or more active substances and at least one inert diluent, such as lactose, mannitol, glucose, microcrystalline cellulose, hydroxypropyl cellulose, starch, polyvinylpyrrolidone or magnesium aluminate metasilicate. The composition may contain additives other than inert diluents, for example lubricants, disintegrators, stabilizers or dissolution agents or auxiliary dissolution agents according to general methods. If necessary, tablets or pills may be coated with films such as sugar coating, or gastric or enteric coatings.
The oral liquid composition may include for example emulsions, solutions, suspensions, syrups or elixirs and may contain inert diluents for general use, for example distilled water or ethanol. The composition may contain additives other than inert diluents, for example, a moistening agent, a suspending agent, sweeteners, a flavoring agent or antiseptic.
Non-parenteral injections may include aseptic, aqueous or non-aqueous solutions, suspensions or emulsions. The aqueous solutions or suspensions may contain for example distilled water for injection or physiological saline as diluents. The diluents for non-aqueous solutions or suspensions include for example propylene glycol, polyethylene glycol, plant oils (for example, olive oil) and alcohols (for example, ethanol), or polysorbate 80. The composition described above may contain a moistening agent, an emulsifying agent, a dispersant, a stabilizer, a dissolution agent or an auxiliary dissolution agent, or antiseptic. The said composition can be sterilized by filtration through bacteria-trapping filters, blending of sterilizing agents or irradiation. Additionally, an aseptic solid composition is produced, which is then dissolved in aseptic water or other aseptic medium for injection prior to use and can be then used.
The dose can be appropriately determined, in view of the intensity of the activity of the active component, namely a substance obtained by the screening method of the present invention, the symptom, and age or sex of a subject for administration.
In case of oral dosing, for example, the dose is about 0.1 to 100 mg, preferably 0.1 to 50 mg per adult (with a body weight of 60 kg) per day. In case of parenteral dosing in the form of an injection, the dose is 0.01 to 50 mg, preferably 0.01 to 10 mg per day.

Examples

The present invention is now described in detail in the following Examples. However, the present invention is not limited by the Examples. Unless otherwise described, the present invention may be carried out according to the known method ("*Molecular Cloning*", Sambrook, J., *et al.,* Cold Spring Harbor Laboratory Press, 1989, *etc*.). In case of using commercially available reagents or kits, the present invention can be carried out according to the instructions attached to the commercially available products.

(Example 1)

Identification of binding protein to the AF-1 of PPARγ

(1) Isolation of PPARγ gene and preparation of plasmid pcDNA-PPARγ for expression in animal cells

Using the primers of SEQ ID NOS:6 and 7, a cDNA fragment of 1518 bp (base pairs) encoding the full-length PPARγ2 was obtained from the human fat tissue cDNA library (Clontech) by PCR [using DNA polymerase (LA Taq DNA polymerase: Takara Shuzo Co., Ltd.) at 94°C (5 minutes) and subsequently 35 times of a cycle of 94°C (30 seconds), 55°C (30 seconds) and 72°C (90 seconds), followed by heating at 72°C for 7 minutes]. The cDNA encoding the full-length PPARγ2 was inserted in an expression vector for animal cells, namely pcDNA3.1/V5-His-TOPO vector (Invitrogen) by the TOPO cloning method (Invitrogen) using in vitro recombination to prepare a plasmid of pcDNA-PPARγ for expression in animal cells.

(2) Preparation of plasmid of pGEX-PPARγ-AF-1 for expressing glutathione S-transferase (GST)-fused protein and expression of GST-PPARγ-AF-1 fusion protein

Using the primers of SEQ ID NOS:6 and 8 and the pcDNA-PPARγ prepared in Example 1 (1) as template for PCR [using DNA polymerase (Taq DNA polymerase; Sigma) at 94°C (5 minutes) and subsequently 25 times of a cycle of 94°C (30 seconds), 55°C (30 seconds) and 72°C (30 seconds), followed by heating at 72°C for 7 minutes], a cDNA fragment of about 600 bp encoding a region including the AF-1 of PPARγ was obtained. This was treated by restriction enzymes (*EcoR*I and *Not*I; Takara Shuzo Co., Ltd.), and inserted in pGEX-6P-1 (Amersham Biosciences) similarly treated with restriction enzymes to prepare a plasmid of pGEX-PPARγ-AF-1 for expressing GST fusion protein. *Escherichia coli* transformed with the plasmid was cultured at 37°C for 3 hours, to which isopropyl-β-D-thioglactopyranoside (IPTG; Nakarai Tesque) was added to become a final concentration of 2.5 mM to induce the expression of the fusion protein, followed by further culturing at 27°C for another 6 hours. Subsequently, the *Escherichia coli* was collected. The cell was disrupted with an ultrasonic generator (201M; Kubota) to prepare GST-PPARγ-AF-1 fusion protein.

(3) GST pull-down assay

After binding the GST-PPARγ-AF-1 fusion protein prepared in Example 1 (2) was bound to a gel (glutathione Sepharose 4B; Amersham Pharmacia), the gel was washed with an appropriate buffer to remove non-specific protein binding. Using an expression vector of p68 RNA helicase, namely pSG5-p68 (Endoh, *et al., Mol. Cell. Biol*. Vol.19, pp. 5363-5372, 1999) as a template and according to the protocol attached to the kit, the *in vitro* protein expression kit (TNT$^R$T7 Quick Coupled Transcription/Translation System; Promega) and the full-length p68 RNA helicase protein which is radio-labeled with radioactive methionine (EASYTAG™ EXPRESS PROTEIN LABELING MIX [$^{35}$S]-; NEN Life Sciences) were mixed with the gel bound with the said GST-PPARγ-AF-1 fusion protein and binding reaction was carried out at 4°C for one hour, followed by washing. This was separated, using sodium dodecyl sulfate-modified polyacrylamide gel (SDS-PAGE), and analyzed by an imaging analyzer (Typhoon 8600; Pharmacia Biosciences). As a result, the binding of the AF-1 of PPARγ to p68 RNA helicase was confirmed. This apparently indicates that p68 RNA helicase is a factor binding to the AF-1 of PPARγ.

(Example 2)

Detection of the regulatory activity of p68 RNA helicase on the transcription induction ability of PPARγ in the presence of ligand

The above results indicate that p68 RNA helicase interacts with the AF-1 of PPARγ. It was examined by reporter assay using cultured cells of COS-1 that what kind of effects p68 RNA helicase had on the transcription induction activity of PPARγ. A thiazolidine derivative reported to act as a ligand of PPARγ, namely pioglitazone [(+)-5-[4-[2-(5-ethyl-2-pyridinyl)ethoxy]benzyl]-2,4-thiazolidinedione; Takeda Pharmaceutical Co., Ltd.: Patent No. 1853588] was synthesized by the method described in the specification.

(1) Detection of the regulatory activity of p68 RNA helicase on the transcription induction ability of PPARγ

Cultured cells of COS-1 were cultured in 100 μl of the minimum essential culture medium DMEM (Gibco) supplemented with 10% fetal bovine serum (Sigma) per well in a 96-well culture plate (Asahi Technoglass) to become 90% confluence. The following substances (A), (B), (C) and (D) were transiently co-transfected in the cell using a lipofection reagent (Lipofectamine 2000; Invitrogen) according to the protocol attached to the lipofection reagent.

(A) pcDNA-PPARγ (30 ng/well) prepared in Example 1 (1)
(B) Reporter construct with the PPAR binding sequence arranged upstream of the luciferase gene (Kliewer, *et al*., Nature, Vol. 358, pp. 771-774, 1992) (100 ng/well)
(C) p68 RNA helicase-expressing vector of pSG5-p68 (Endoh, *et al*., *Mol. Cell. Biol*., Vol.19, pp. 5363-5372, 1999) (0-10 ng/well)
(D) Plasmid of pCMV-β-galactosidase control vector with the gene expressing β-galactosidase (Roche Diagnostics) 10 ng/well

After adding pioglitazone which is a PPARγ agonist to the co-transfected cell to become a final concentration of 30 nM, the cell was cultured for 24 hours. The culture medium was discarded and the cell was washed with phosphate buffered saline(PBS). Per each well, 80 μl each of lysis solution (100 mM potassium phosphate, pH 7.8, 0.2% Triton X-100) was added, for cell lysis. To 20 μl of the cytolytic solution, 100 μl of a luciferase substrate solution (Wako Pure Chemical Co., Ltd.) was added to assay the luminescence with a chemiluminescence meter (Type ML3000; Dynatech Laboratories). Separately, the β-galactosidase activity of the cytolytic solution was assayed with a kit for detecting the β-galactosidase activity (Galacto-Light Plus™ system; TROPIX), and the resulting value was digitized. This was used as the transfection efficiency of introduced gene, to correct the said luciferase activity per each well.

As the results of the experiments, the agonist-dependent transcription induction activity of PPARγ was promoted depending on the amount of p68 RNA helicase by the co-expression of p68 RNA helicase (Fig.1). The fact shows that p68 RNA helicase is one of transcriptional coactivators of PPARγ. Using the finding, it is possible to decrease the blood glucose level through the increase of the amount of p68 RNA helicase by directing biological energy sources toward glucose metabolism. In other words, by the increase of the amount of p68 RNA helicase, transcription induction ability of PPARγ was promoted. As a result, activity similar to PPARγ agonist, namely activity of improving insulin resistance can be expected. By the experimental system, the detection and/or screening of a substance promoting the transcription induction activity of PPARγ and a substance improving insulin resistance are enabled.

(Example 3)

Confirmation of expression of p68 RNA helicase in human tissue

Using primers of SEQ ID NOS:9 and 10 and according to PCR [using DNA polymerase (Taq DNA polymerase; Sigma) at 94°C (5 minutes) and subsequently 35 times of a cycle of 94°C (30 seconds), 55°C (30 seconds) and 72°C (90 seconds), followed by heating at 72°C for 7 minutes], the amplification of a cDNA fragment of about 800 bp encoding p68 RNA helicase in the human cDNA library (Clontech) was detected by agarose gel electrophoresis. Consequently, it was found that p68 RNA helicase was expressed in fat tissue and muscle known to have the PPARγ activity. This was supported even on the basis of the expression sites that p68 RNA helicase was a transcriptional coactivator of PPARγ.

(Example 4)

Comparison of the expression level of mRNA of p68 RNA helicase during the differentiation course of 3T3L1 cell into fat cell

To a culture plate (60-mm diameter; Asahi Technoglass), 2 ml of the minimum essential culture medium of DMEM (Gibco) supplemented with 10% fetal bovine serum (Sigma) was, added and a culture cell 3T3L1 cell was cultured therein to become confluence. Subsequently, the culture medium was replaced with a differentiation culture medium [the minimum essential culture medium DMEM (Gibco) supplemented with insulin (at a final concentration of 10 μg/ml; Sigma), dexamethazone (at a final concentration of 250 μM; Sigma) and 3-isobutyl-1-methoxylxanthine (at a final concentration of 500 μM; Sigma)], to which pioglitazone (at a final concentration of 1 μM) as an agent for improving insulin resistance was added or was not added. Then, it was examined as to whether or not the expression level of p68 RNA helicase changed between the culture medium with or without adding pioglitazone. 24 hours after pioglitazone addition, the cell was harvested, to extract RNA using an RNA extraction reagent (ISOGEN; Wako Pure Chemical Co., Ltd.) for reverse transcription with a reverse transcription reaction kit (Thermoscript RT-PCR System; Invitrogen). Using the resulting product as template and additionally using a primer set represented by SEQ In NOS: 11 and 12 (p68 RNA helicase) or by SEQ ID NOS:13 and 14 (G3PDH) and a detection reagent (2X SYBR Green Master Mix; Applied Biosystems), the changes of the expression levels of p68 RNA helicase and G3PDH were examined by the real-time PCR (Prism 7700 Sequence Detection System; Applied Biosystems). The expression level of the p68 RNA helicase gene was corrected on the basis of the expression level of the G3PDH gene by the following formula.

[Corrected expression level of p68 RNA helicase] =

[expression level of p68 RNA helicase (raw data)] /

[expression level of G3PDH (raw data)]

As a result, compared with the culture medium without pioglitazone addition, the expression level of p68 RNA helicase in the culture medium with pioglitazone addition was about 1.7 fold. This supported that pioglitazone which is an agent for improving insulin resistance had an activity of increasing the expression level of p68 RNA helicase and that the increase of the expression of p68 RNA helicase improves insulin resistance.

(Example 5)

Detection of promoter activity of p68 RNA helicase gene

(1) Isolation of promoter region of p68 RNA helicase gene and preparation of reporter vector

Using primers represented by SEQ ID NOS:14 and 15 designed on the basis of the nucleotide sequence of the promoter of p68 RNA helicase gene as previously reported (Rbssler, *et al*., *Nucleic Acids Res.,* Vol.28, pp. 932-939, 2000) and the human genome DNA sequence (GenBank accession No. AC009994) and additionally using the human genome DNA (Clontech) as template for PCR [using DNA polymerase (LA Taq DNA polymerase; Takara Shuzo Co., Ltd.), and at 98°C (5 minutes) and subsequently 35 times of a cycle of 96°C (30 seconds), 55°C (30 seconds) and 72°C (90 seconds), followed by heating at 72°C for 7 minutes], a DNA fragment comprising the promoter region of p68 RNA helicase gene represented by SEQ ID NO.5 was obtained. The DNA fragment was treated with restriction enzymes (*Kpn*I and *Xho*I; Takara Shuzo Co., Ltd.), to be ligated to the luciferase reporter vector similarly treated with restriction enzymes (pGL3-Basic vector; Promega). Thus, a p68 RNA helicase gene promoter-fused reporter vector (pGL3-p68-1184bp) was constructed. Further, the pGL3-p68-1184bp was treated with restriction enzymes (*Nhe*I and *Xho*I; Takara Shuzo Co., Ltd.), to obtain a DNA fragment comprising the promoter region of p68 RNA helicase gene up to the -899 bp. The DNA fragment was then ligated to the pGL3-Basic vector similarly treated with restriction enzymes, to construct a p68 RNA helicase gene promoter-fused reporter vector (pGL3-p68-899bp).

(2) Detection of promoter activity of p68 RNA helicase gene

pGL3-p68-899bp, pGL3-p68-1184bp constructed in Example 5(1) or pGL3-Basic as a negative control (100 ng/well) was individually transiently co-transfected together with a β-galactosidase-expression vector (pCMV-β-galactosidase control vector; Roche Diagnostics)(10 ng/well) into the COS-1 cells. Co-transfection was carried out by the same method as in Example 2 (1). After culturing for 48 hours, the luminescence of luciferase was assayed in the same manner as in Example 2. As in Example 2, the β-galactosidase activity was assayed and used as the transfection efficiency of the introduced gene to correct the luciferase activity per each well.

As the results of the experiments, the promoter activity of p68 RNA helicase gene was very strong compared with the negative control pGL3-Basic (about 202 fold in case of pGL3-p68-1184bp and about 94 fold in case of pGL3-p68-899bp). Since the activity obtained with pGL3-p68-1184 bp was about half the activity obtained with pGL3-p68-899 bp, it was shown that the region from -1184 bp to -899 bp was involved in the suppressive regulation of transcription. Thus, it was expected that the substance with an activity of removing the suppressive regulation would consequently have an activity of promoting the transcription of p68 RNA helicase gene. Therefore, such substance can be screened for according to the present invention using pGL3-p68-1184bp.

(3) Detection of regulatory activity of an agent for improving insulin resistance on promoter activity of p68 RNA helicase gene

After adding pioglitazone as one of agents for improving insulin resistance to the co-transfected cell in Example 5 (2) to a final concentration of 10 μM, and culturing for 24 hours, the luciferase activity was assayed in the same manner as in Example 2. As in Example 2, the β-galactosidase activity was assayed to correct the said luciferase activity per each well using the β-galactosidase activity as the transfection efficiency of the introduced gene.

As the result of the experiments, the promoter activity of p68 RNA helicase gene was activated depending on the agent for improving insulin resistance (Fig.2). The fact demonstrates that the transcription of p68 RNA helicase gene is activated by pioglitazone as one of agents for improving insulin resistance and that the mechanism of improving insulin resistance is the activation of the transcription of p68 RNA helicase gene.

Additionally, since the activation of the promoter activity of p68 RNA helicase gene with pioglitazone as one of agents for improving insulin resistance is observed with both pGL3-p68-899bp and pGL3-p68-1184bp, it is considered that the function point for the transcription activation with pioglitazone exists in 3'-downstream of the -899 bp. Accordingly, the activation is not the removal of the transcription suppressive regulation in the region from -1184 bp to -899 bp. Thus, screening a substance with an activity of removing the suppressive regulation of p68 RNA helicase gene, more specifically a substance activating the reporter activity of pGL3-p68-1184bp enabled the detection of or screening a substance inducing the expression of p68 RNA helicase and improving insulin resistance, which is different from a conventional agent for improving insulin resistance.

These supported a possibility of improving insulin resistance by the increased expression of p68 RNA helicase. When the expression of p68 RNA helicase is increased using this, biological energy sources can be directed toward glucose metabolism to decrease blood glucose value. The experimental system enabled the detection of and/or screening a substance improving insulin resistance.

## Industrial Applicability

The screening system using the interaction between PPARγ and the PPAR-interactive p68 RNA helicase and the screening system using the induction of the PPAR-interactive p68 RNA helicase expression can be used for screening a new type of a pharmaceutical agent which is different from PPARγ synthetic ligands as a conventional agent for improving insulin resistance. The cell of the present invention can be used for constructing said screening system.

Additionally, a pharmaceutical composition for improving insulin resistance can be produced by using a substance obtainable by the screening method of the present invention as the active ingredient and using a carrier, an excipient and/or other additives for preparation.

## Free Text of Sequence Listings

In the numerical title [223] in the Sequence Listing below, the [Artificial Sequence] is described. Specifically, individual nucleotide sequences of SEQ ID NOS.:6-8, 10, 14, and 15 in the Sequence Listing are primer sequences artificially synthesized.

Although the present invention has been described hereinabove with the reference to the specific embodiments, variations and modifications thereof obvious to persons skilled in the art are also included within the scope of the present invention.

## Claims

1. A cell transformed by

   i) a polynucleotide encoding a polypeptide which comprises an amino acid sequence represented by SEQ ID NO:2 in which 1 to 10 amino acids are deleted, substituted and/or inserted and which interacts with PPARγ,
   ii) a polynucleotide encoding a fusion protein comprising at least the AF-1 of the PPARγ protein represented by SEQ ID NO:4 and the DNA binding domain of a transcription factor, and
   iii) a reporter gene fused to a response element to which the DNA binding domain of said transcription factor can bind; or

   a cell transformed by

   i) a polynucleotide encoding a polypeptide comprising an amino acid sequence represented by SEQ ID NO: 2 in which 1 to 10 amino acids are deleted, substituted and/or inserted and which interacts with PPARγ and
   ii) a reporter gene fused to a response element to which the PPARγ protein represented by SEQ ID NO:4 is able to bind, and expressing

   a) a polypeptide comprising a protein consisting of an amino acid sequence represented by SEQ ID NO: 2 in which 1 to 10 amino acids are deleted, substituted, and/or inserted, and which interacts with PPARγ and b) the PPARγ protein represented by SEQ ID NO:4.

2. The cell according to claim 1, wherein the transcription factor is a yeast GAL4 protein.

3. The cell according to claim 1, wherein the reporter gene is a luciferase gene.

4. A method for detecting whether or not a test substance promotes the transcription induction activity of PPARγ, comprising

   i) a step of allowing the cell according to one of claims 1 to 3 to contact with the test substance, and
   ii) a step of analyzing the change of the test substance-dependent interaction or the change of the test substance-dependent transcription induction activity of PPARγ, in which expression of the reporter gene is used as an index.

5. A method for screening a substance promoting the transcription induction activity of PPARγ, comprising

   (i) a step of allowing the cell according to one of claims 1 to 3 to contact with a test substance,
   ii) a step of analyzing the change of the test substance-dependent interaction or the change of the test substance-dependent transcription induction activity of PPARγ, in which expression of the reporter gene is used as an index and
   iii) a step of selecting a test substance which activates the reporter activity.

**6.** The method for screening according to claim 5, wherein the substance promoting the transcription induction activity of PPARγ is an agent for improving insulin resistance.

**7.** A method for screening an agent for improving insulin resistance, comprising

i) a step of allowing a cell expressing PPAR-interactive p68 RNA helicase to contact with a test substance, and
ii) a step of analyzing the change of the test substance-dependent expression level of PPAR-interactive p68 RNA helicase.

**8.** A screening method for an agent for improving insulin resistance, comprising

i) a step of allowing a cell transformed with a reporter gene fused with the promoter region of p68 RNA helicase represented by SEQ ID NO:5 to contact with a test substance, and
ii) a step of analyzing the change of the test substance-dependent transcription induction activity, in which the expression of the reporter gene is used as an index.

**9.** A method for producing a pharmaceutical composition for improving insulin resistance, comprising
a screening step using the screening method according to one of claims 5 to 8, and
a formulation step using a substance obtainable by said screening.

*FIG. 1*

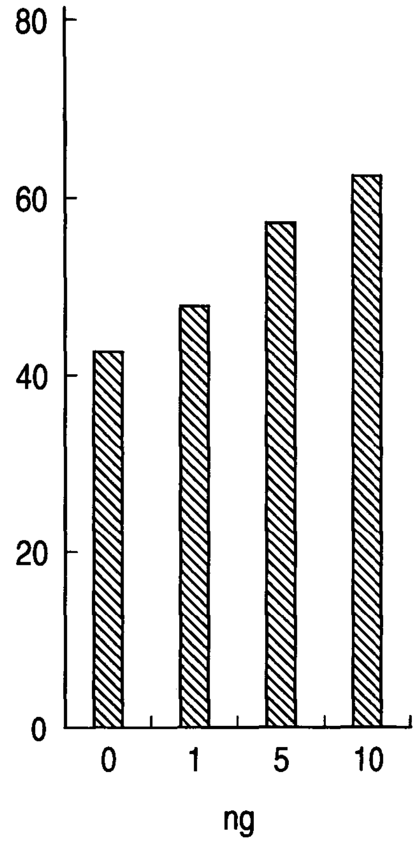
80
60
40
20
0
0
1
5
10
ng

*FIG. 2*

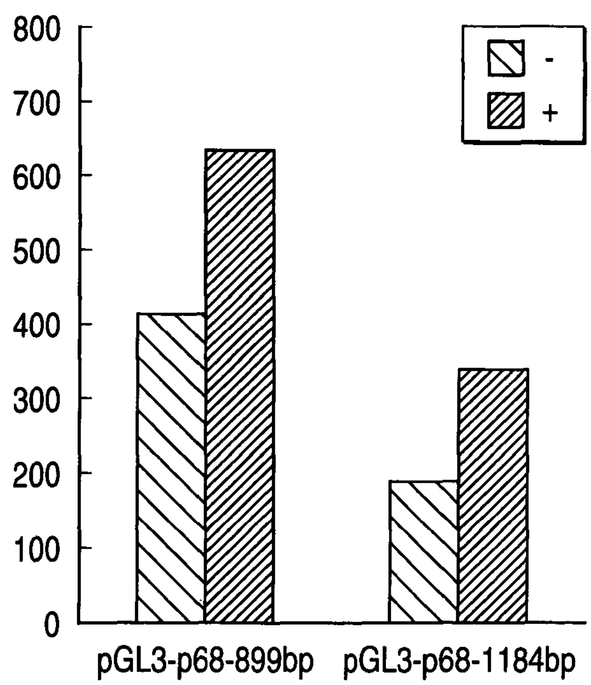
800
700
600
500
400
300
200
100
0
-
+
pGL3-p68-899bp
pGL3-p68-1184bp

## INTERNATIONAL SEARCH REPORT

International application No.
PCT/JP03/08367

A. CLASSIFICATION OF SUBJECT MATTER
Int.Cl[7] C12N5/10, C12N15/09, C12Q1/02, A61K45/00, A61P3/08

According to International Patent Classification (IPC) or to both national classification and IPC

B. FIELDS SEARCHED
Minimum documentation searched (classification system followed by classification symbols)
Int.Cl[7] C12N5/10, C12N15/09, C12Q1/02, A61K45/00, A61P3/08

Documentation searched other than minimum documentation to the extent that such documents are included in the fields searched

Electronic data base consulted during the international search (name of data base and, where practicable, search terms used)
BIOSIS(DIALOG), WPI(DIALOG), REGISTRY(STN), CA(STN)

C. DOCUMENTS CONSIDERED TO BE RELEVANT

| Category* | Citation of document, with indication, where appropriate, of the relevant passages | Relevant to claim No. |
|---|---|---|
| A | Endoh H. et al., Purification and identification of p68 RNA helicase acting as a transcriptional coactivator specific for the activation function 1 of human estrogen receptor α., Mol.Cell.Biol., 1999, Vol.19, No.8, pages 5363 to 5372 | 1-8 |
| A | Deeb SS. et al., A Pro12Ala substitution in PPARgamma2 associated with decreased receptor activity, lower body mass index and improved insulin sensitivity., Nat.Genet., 1998, Vol.20, No.3, pages 284 to 287 | 1-8 |

[X] Further documents are listed in the continuation of Box C. [ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 16 September, 2003 (16.09.03) | 30 September, 2003 (30.09.03) |

| Name and mailing address of the ISA/ | Authorized officer |
|---|---|
| Japanese Patent Office | |
| Facsimile No. | Telephone No. |

Form PCT/ISA/210 (second sheet) (July 1998)

**INTERNATIONAL SEARCH REPORT**

International application No.
PCT/JP03/08367

C (Continuation). DOCUMENTS CONSIDERED TO BE RELEVANT

| Category* | Citation of document, with indication, where appropriate, of the relevant passages | Relevant to claim No. |
|---|---|---|
| A | Pihlajamaki J. et al., The Pro12Ala substitution in the peroxisome proliferator activated receptor gamma 2 is associated with an insulin-sensitive phenotype in families with familial combined hyperlipidemia and in nondiabetic elderly subjects with dyslipidemia., Atherosclerosis., 2000, Vol.151, No.2, pages 567 to 574 | 1-8 |
| A | Meirchaeghe A. et al., Impact of the Peroxisome Proliferator Activated Receptor gamma2 Pro12Ala polymorphism on adiposity, lipids and non-insulin-dependent diabetes mellitus., Int.J.Obes.Relat. Metab.Disord., 2000, Vol.24, No.2, pages 195 to 199 | 1-8 |
| A | Patrone C. et al., Divergent pathways regulate ligand-independent activation of ER alpha in SK-N-BE neuroblastoma and COS-1 renal carcinoma cells., Mol.Endocrinol., 1998, Vol.12, No.6, pages 835 to 841 | 1-8 |
| A | WO 01/42307 A (SUMITOMO CHEM. CO., LTD.), 14 June, 2001 (14.06.01), & AU 200118868 A & EP 1237925 A1 & JP 2003-516137 A | 1-8 |
| A | Suk K. et al., Identification of a novel human member of the DEAD box protein family., Biochim. Biophys.Acta., 2000, Vol.1501, No.1, pages 63 to 69 | 1-8 |

Form PCT/ISA/210 (continuation of second sheet) (July 1998)

**INTERNATIONAL SEARCH REPORT**

International application No.
PCT/JP03/08367

**Box I Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☒ Claims Nos.: 9
because they relate to parts of the international application that do not comply with the prescribed requirements to such an extent that no meaningful international search can be carried out, specifically:
Concerning "a substance obtained by the above screening method" as described in claim 9, no specific substance is presented in the description as a substance obtained by the screening method. Thus, claim 9 is neither supported by the description nor disclosed (continued to extra sheet)

3. ☐ Claims Nos.:
because they are dependent claims and are not drafted in accordance with the second and third sentences of Rule 6.4(a).

**Box II Observations where unity of invention is lacking (Continuation of item 3 of first sheet)**

This International Searching Authority found multiple inventions in this international application, as follows:

1. ☐ As all required additional search fees were timely paid by the applicant, this international search report covers all searchable claims.

2. ☐ As all searchable claims could be searched without effort justifying an additional fee, this Authority did not invite payment of any additional fee.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest** ☐ The additional search fees were accompanied by the applicant's protest.
☐ No protest accompanied the payment of additional search fees.

Form PCT/ISA/210 (continuation of first sheet (1)) (July 1998)

**INTERNATIONAL SEARCH REPORT**

International application No.
PCT/JP03/08367

Continuation of Box No.I-2 of continuation of first sheet(1)

therein. Even though the common technical knowledge at the point of the application is taken into consideration, it is completely unknown what specific compounds are involved therein and what are not. Thus, the above claim is described in an unclear manner. Such being the case, no meaningful search can be made on the invention as set forth in the above claim.

Form PCT/ISA/210 (extra sheet) (July 1998)